# EUROPEAN PATENT APPLICATION

(11) **EP 4 176 889 A1**
(43) Date of publication of application: **10.05.2023**
(21) Application number: 21808009.1
(22) Date of filing: 19.05.2021
(51) Int. Cl.: A61K 35/745, A23L 33/135, A61K 31/702, A61P 1/00, A61P 11/00, A61P 37/00, A23C 9/152, A23C 9/18

(54) **COMPOSITION FOR PROMOTING INTESTINAL TRACT DEVELOPMENT, COMPOSITION FOR IMPROVING PULMONARY FUNCTION AND COMPOSITION FOR ENHANCING IMMUNE FUNCTION**

(30) Priority: 22.05.2020 JP 2020089635; 22.05.2020 JP 2020089636; 22.05.2020 JP 2020089637
(71) Applicant: Morinaga Milk Industry Co., Ltd., Minato-ku Tokyo 108-8384 (JP)
(72) Inventor: HIRAKU, Akari, Zama-shi, Kanagawa 252-8583 (JP); IWABUCHI, Noriyuki, Zama-shi, Kanagawa 252-8583 (JP); SUDA, Yoshihito, Sendai-shi, Miyagi 982-0215 (JP)
(74) Representative: Hoffmann Eitle
(86) International application number: PCT/JP2021/018987
(87) International publication number: WO 2021/235486

(57) **Abstract**

A composition for promoting development of the intestinal tract, a composition for improving lung function or a composition for improving immune function containing a bacterium belonging to *Bifidobacterium longum* subsp. *infantis* is provided. The bacterium is preferably *Bifidobacterium longum* subsp. *infantis* M-63 (NITE BP-02623) .

## Description

### [Technical Field]

The present invention relates to a composition for promoting development intestinal tract development and a composition for preventing or treating an intestinal disease or relieving a symptom thereof. The invention relates to a composition for improving lung function and a composition for preventing or treating a lung disease or relieving a symptom thereof. The invention relates to a composition for improving immune function.

### [Background Art]

The gastrointestinal tract is a long tube from the mouth to the anus and divided into oral cavity, pharynx, esophagus, stomach, small intestine (duodenum, jejunum and ileum) and large intestine (cecum, appendix, ascending colon, transverse colon, descending colon, sigmoid colon and rectum). The intestinal tract including the small intestine and the large intestine has the function of digesting food and absorbing nutrients. Normal development of the intestinal tract is important for the growth of an individual and the maintenance of health.

Some of bifidobacteria and lactic acid bacteria are known to regulate the microbiome when being taken and to contribute to the human health. For example, patent literature 1 (JP-A-2014-111668) discloses that an oral composition containing *Lactobacillus brevis* and lactoferrin as active ingredients improves the defecation frequency and the amount of defecation.

Pneumonia is a disease which causes inflammation of the lungs due to infection with a pathogenic microorganism such as bacteria and viruses. Pneumonia is particularly common in children under the age of 18 years and in adults of the age of 75 years or older and is a major cause of death among newborns, infants and small children. Typical symptoms of pneumonia are fever, cough, sputum, respiratory distress, general malaise, chest pain and the like. Lung diseases such as pneumonia cause a decrease in lung function.

The intestinal tract is known to have barrier function for preventing entry of substances which are foreign substances for the body and serve as an immunological organ for protecting the body from pathogenic bacteria. It is said that 70% of the immune cells of the whole body are concentrated in the intestines, and the intestines play an important role in the health of the whole body.

The possibility that some lactic acid bacteria involve in immune function has been suggested. PTL 2 (JP-A-2012-158568) discloses that lactic acid bacteria inhibit inflammation of joints. PTL 3 (JP-A-2018-177740) discloses that metabolites of lactic acid bacteria regulate the cytokine production.

### [Citation List]

### [Patent Literature]

[PTL 1] JP-A-2014-111668
[PTL 2] JP-A-2012-158568
[PTL 3] JP-A-2018-177740

### [Summary of Invention]

### [Problems to be Solved by the Invention]

An object of the invention is to provide a novel composition which promotes development of the intestinal tract and a novel composition which prevents or treats an intestinal disease or which relieves a symptom thereof. Another object of the invention is to provide a novel composition which improves lung function and a novel composition which prevents or treats a lung disease or which relieves a symptom thereof. Another object of the invention is to provide a novel composition which improves immune function.

### [Solution to Problem]

The invention relates to [1] to [14] exemplified below.
[1] A composition for promoting development of the intestinal tract containing a bacterium belonging to *Bifidobacterium longum* subsp. *infantis.*
[2] The composition described in [1] which promotes formation of a fold in the intestinal tract.
[3] A composition for preventing or treating an intestinal disease or relieving a symptom thereof containing a bacterium belonging to *Bifidobacterium longum* subsp. *infantis.*
[4] The composition described in [3], wherein the intestinal disease is a disease caused from a failure of development of the intestinal tract or a decrease in intestinal tract function.
[5] The composition described in any of [1] to [4] which is a food or drink composition.
[6] The composition described in any of [1] to [5], wherein the bacterium is *Bifidobacterium longum* subsp. *infantis* M-63 (NITE BP-02623) .
[7] The composition described in any of [1] to [6], wherein the bacterium is a living bacterium.
[8] The composition described in any of [1] to [7] which further contains a human milk oligosaccharide.
[9] The composition described in any of [1] to [8] which is a modified powder milk or a modified liquid milk.
[10] An agent for promoting development of the intestinal tract or an agent for preventing or treating an intestinal disease or relieving a symptom thereof containing a bacterium belonging to *Bifidobacterium longum* subsp. *infantis.*
[11] Use of a bacterium belonging to *Bifidobacterium longum* subsp. *infantis* for the promotion of development of the intestinal tract or for the prevention or the treatment of an intestinal disease or the relief of a symptom thereof.
[12] A bacterium belonging to *Bifidobacterium longum* subsp. *infantis* used for promoting development of the intestinal tract or for preventing or treating an intestinal disease or relieving a symptom thereof.
[13] Use of a bacterium belonging to *Bifidobacterium longum* subsp. *infantis* in the manufacture of a composition for promoting development of the intestinal tract or a composition for preventing or treating an intestinal disease or relieving a symptom thereof.
[14] A method for promoting development of the intestinal tract or a method for preventing or treating an intestinal disease or relieving a symptom thereof, including a step of administering a bacterium belonging to *Bifidobacterium longum* subsp. *infantis* to a mammal.
   The invention also relates to [15] to [27] exemplified below.
[15] A composition for improving lung function containing a bacterium belonging to *Bifidobacterium longum* subsp. *infantis.*
[16] A composition for preventing or treating a lung disease or relieving a symptom thereof containing a bacterium belonging to *Bifidobacterium longum* subsp. *infantis.*
[17] The composition described in [16], wherein the lung disease is pneumonia.
[18] The composition described in any of [15] to [17] which is a food or drink composition.
[19] The composition described in any of [15] to [18], wherein the bacterium is *Bifidobacterium longum* subsp. *infantis* M-63 (NITE BP-02623) .
[20] The composition described in any of [15] to [19], wherein the bacterium is a living bacterium.
[21] The composition described in any of [15] to [20] which further contains a human milk oligosaccharide.
[22] The composition described in any of [15] to [21] which is a modified powder milk or a modified liquid milk.
[23] An agent for improving lung function or an agent for preventing or treating a lung disease or relieving a symptom thereof containing a bacterium belonging to *Bifidobacterium longum* subsp. *infantis.*
[24] Use of a bacterium belonging to *Bifidobacterium longum* subsp. *infantis* for the improvement of lung function or for the prevention or the treatment of a lung disease or the relief of a symptom thereof.
[25] A bacterium belonging to *Bifidobacterium longum* subsp. *infantis* used for improving lung function or for preventing or treating a lung disease or relieving a symptom thereof.
[26] Use of a bacterium belonging to *Bifidobacterium longum* subsp. *infantis* in the manufacture of a composition for improving lung function or a composition for preventing or treating a lung disease or relieving a symptom thereof.
[27] A method for improving lung function or a method for preventing or treating a lung disease or relieving a symptom thereof, including a step of administering a bacterium belonging to *Bifidobacterium longum* subsp. *infantis* to a mammal.
   The invention also relates to [28] to [41] exemplified below.
[28] A composition for improving immune function containing a bacterium belonging to *Bifidobacterium longum* subsp. *infantis.*
[29] The composition described in [28] which regulates production of a cytokine.
[30] The composition described in [29], wherein the cytokine includes at least one selected from the group consisting of interleukin-6, interleukin-8, monocyte chemotactic protein, interferon-β and interferon-γ.
[31] The composition described in [28] which inhibits infection with a pathogenic microorganism.
[32] The composition described in any of [28] to [31] which is a food or drink composition.
[33] The composition described in any of [28] to [32], wherein the bacterium is *Bifidobacterium longum* subsp. *infantis* M-63 (NITE BP-02623).
[34] The composition described in any of [28] to [33], wherein the bacterium is a living bacterium.
[35] The composition described in any of [28] to [34] which further contains a human milk oligosaccharide.
[36] The composition described in any of [28] to [35] which is a modified powder milk or a modified liquid milk.
[37] An agent for improving immune function containing a bacterium belonging to *Bifidobacterium longum* subsp. *infantis.*
[38] Use of a bacterium belonging to *Bifidobacterium longum* subsp. *infantis* for the improvement of immune function.
[39] A bacterium belonging to *Bifidobacterium longum* subsp. *infantis* used for improving immune function.
[40] Use of a bacterium belonging to *Bifidobacterium longum* subsp. *infantis* in the manufacture of a composition for improving immune function.
[41] A method for improving immune function, including a step of administering a bacterium belonging to *Bifidobacterium longum* subsp. *infantis* to a mammal.

### [Effects of The Invention]

According to the invention, a novel composition which promotes development of the intestinal tract, a novel composition which prevents or treats an intestinal disease or which relieves a symptom thereof, a novel composition which improves lung function, a novel composition which prevents or treats a lung disease or which relieves a symptom thereof and a novel composition which improves immune function can be provided.

### [Brief Description of Figures]

[Fig. 1] Pictures of lumina of the intestinal tract of a pig of the M-63 administration group in Experiment 1.
[Fig. 2] Pictures of lumina of the intestinal tract of a pig of the control group in Experiment 1.
[Fig. 3] A picture of the right middle lobe and the posterior lobe of the abdominal side of a lung of a pig of the M-63 administration group in Experiment 2.
[Fig. 4] A picture of the right middle lobe and the posterior lobe of the abdominal side of a lung of a pig of the control group in Experiment 2.
[Fig. 5] A CD8 immunostaining image of the large intestine of a pig of the M-63 administration group in Experiment 3.
[Fig. 6] A CD8 immunostaining image of the large intestine of a pig of the control group in Experiment 3.

### [Description of Embodiments]

### <Agent for Promoting Development of Intestinal Tract and Composition for Promoting Development of Intestinal Tract>

The agent for promoting development of the intestinal tract according to the invention contains a bacterium belonging to *Bifidobacterium longum* subsp. *infantis.* The agent for promoting development of the intestinal tract according to the invention can promote development of the intestinal tract of a subject of the intake or the administration.

The composition according to an embodiment of the invention is a composition for promoting development of the intestinal tract and contains a bacterium belonging to *Bifidobacterium longum* subsp. *infantis.* The composition for promoting development of the intestinal tract according to the invention may contain the agent for promoting development of the intestinal tract according to the invention or may be the agent for promoting development of the intestinal tract according to the invention itself. The composition for promoting development of the intestinal tract according to the invention can promote development of the intestinal tract of a subject of the intake or the administration.

In the present specification, the intestinal tract includes the small intestine and the large intestine. The intestinal tract is an organ responsible for the digestion of food and the absorption of nutrients. The small intestine includes the duodenum, the jejunum and the ileum. Digestive enzymes are secreted from the mucosal layer of the small intestine, and food digested in the stomach is digested into the final decomposition products such as amino acids, glucose, glycerides and fatty acids. The small intestine repeats contraction and relaxation and moves and absorbs the mixture of the food and the digestive juice. This movement helps mixing the digestive juice and the food and at the same time increases the contact between the food and the mucosa and improves the absorption efficiency. The small intestine is composed of four layers, namely the mucosa, the submucosa, the muscularis and the serosa, from the inside. Many folds (circular folds) are in the lumen of the small intestine and surround the lumen, and countless villi are on the surface of the folds, which contributes to the increase in the surface area of the mucosa of the small intestine. The circular folds are composed of the mucosa and the submucosal tissues. The small intestine also has a function as an immunological organ, and aggregated lymphoid nodules (Peyer's patches) are scattered on the inner surface of the small intestine. In the Peyer's patches, major immune cells such as dendritic cells, T cells and B cells are concentrated. The intestinal tract immune cell group produces immunoglobulin A against invading pathogenic bacterial antigens and functions to prevent entry of the pathogenic bacteria into the body through the intestinal tract walls.

The large intestine includes the cecum, the appendix, the ascending colon, the transverse colon, the descending colon, the sigmoid colon and the rectum. The large intestine absorbs water and salts and moves the contents towards the rectum through peristaltic movement. The large intestine secretes mucus, and the large intestine is composed of four layers, namely the mucosa, the submucosa, the muscularis and the serosa, from the inside. The large intestine has three taeniae coli (omental taenia coli, mesocolic taenia coli and free taenia coli) which run from the cecum to the sigmoid colon and which are composed of longitudinal muscles. Through contraction of the taeniae coli, folds (meniscal folds) are created in the lumen of the large intestine. Many enteric bacteria live in the large intestine, which has a low oxygen concentration, and form the enteric microbiota. The enteric microbiota is known to greatly affect the health of the host. Symptoms and diseases affected by the enteric microbiota include constipation, diarrhea, irritable bowel syndrome, inflammatory bowel diseases, metabolic disorder, infections, food poisoning, allergies, malignant tumors including colon cancer and the like.

That the intestinal tract develops means that the intestinal tract has the feature for better carrying out the above function of the intestinal tract. That the intestinal tract develops includes that the intestinal tract has the structural feature for better carrying out the function of digesting food and absorbing nutrients in the intestinal tract. Specifically, the development of the intestinal tract includes formation of folds of the intestinal tract (especially formation of circular folds in the small intestine), formation of villi, secretion of mucus, development of smooth muscles (longitudinal muscle and circular muscle), regulation of peristaltic movement, regulation of secretion of gastrointestinal hormones, development of the intrinsic nervous system which regulates peristaltic movement, hormone secretion or the like, regulation of secretion of digestive juice, formation of the enteric microbiota and the like. These can affect each other. For example, when the formation of folds of the intestinal tract is not sufficient, the surface area of the intestinal tract decreases, and thus the number of cells in the lumen side of the intestinal tract also decreases, which may cause, for example, a decrease or insufficient development of intestinal tract epithelial cells, which absorb nutrients, goblet cells, which secrete mucus, immune cells, enteric bacteria and the like. Promotion of the development of the intestinal tract contributes to growth of an individual, an increase in the body weight, maintenance of excellent nutritional status, health promotion, improvement of the defecation frequency and the defecation amount, enhancement of the barrier function of the intestinal tract walls, improvement of the immune function of the intestinal tract and prevention of allergies and can exert an influence on the brain such as brain-gut interaction (reduction in anxiety and improvement of cognitive function/inhibition of its deterioration).

In mammals, the alimentation system shifts from the transplacental system to the enteral system after birth. A newborn starts taking the breast milk shortly after birth, takes nutrients from the breast milk until weaning and shifts to solid foods after weaning. With the shift of the alimentation form, the form of the intestinal tract and the intestinal mucosa and the digestion-absorption mechanism of nutrients change. Because the bodies of infants and small children are immature, promotion of development of the intestinal tract in the infancy or the early childhood is important for the growth of the infants and the small children. Moreover, because the development of the intestinal tract also helps the improvement of the immunocompetence, development of the intestinal tract is important for infants and small children whose immune function is insufficient.

In this regard, one of the causes for insufficient development of the intestinal tract is inflammation of the intestines. In particular, when inflammation of an intestine is caused in an infant or a small child, it is believed that the formation of folds of the intestinal tract, the formation of villi and the like tend to be underdeveloped.

### <Agent for Preventing or Treating Intestinal Disease or Relieving Symptom Thereof and Composition for Preventing or Treating Intestinal Disease or Relieving Symptom Thereof>

The agent for preventing or treating an intestinal disease or relieving a symptom thereof according to the invention contains a bacterium belonging to *Bifidobacterium longum* subsp. *infantis.* The agent for preventing or treating an intestinal disease or relieving a symptom thereof according to the invention can prevent or treat an intestinal disease or relieve a symptom thereof in a subject of the intake or the administration.

The composition according to an embodiment of the invention is a composition for preventing or treating an intestinal disease or relieving a symptom thereof and contains a bacterium belonging to *Bifidobacterium longum* subsp. *infantis.* The composition for preventing or treating an intestinal disease or relieving a symptom thereof according to the invention may contain the agent for preventing or treating an intestinal disease or relieving a symptom thereof according to the invention or may be the agent for preventing or treating an intestinal disease or relieving a symptom thereof according to the invention itself. The composition for preventing or treating an intestinal disease or relieving a symptom thereof according to the invention can prevent or treat an intestinal disease or relieve a symptom thereof in a subject of the intake or the administration. The relief of a symptom includes relief of a symptom accompanying the disease, delay in progress of the disease and reduction in the period required for the recovery of the condition.

In the present specification, the intestinal disease means a disease in the small intestine or the large intestine. The disease of the small intestine is not particularly limited, but examples thereof include small intestinal tumor, gastrointestinal hemorrhage, Crohn's disease, intussusception, gastrointestinal stromal tumor, malabsorption syndrome, Behcet's disease and the like. The disease of the large intestine is not particularly limited, but examples thereof include colon cancer, polyps, colonic diverticulitis, acute appendicitis, irritable bowel syndrome, ulcerative colitis, ischemic colitis, Crohn's inflammation and the like.

The intestinal disease is a disease caused, for example, from a failure of development of the intestinal tract or a decrease in the intestinal tract function and includes diseases caused when the development or the function of the intestinal tract above is insufficient. Such intestinal diseases include irritable bowel syndrome, inflammatory bowel diseases, metabolic disorder, infections, food poisoning, allergies, malignant tumors including colon cancer and the like. Inflammatory bowel diseases include inflammation caused by external factors, such as infection with microorganisms including bacteria, injury, physical stimulation by heat, coldness, radiation, drugs or the like and chemical substances, as well as Crohn's disease, ulcerative colitis, necrotizing enterocolitis, Behcet's disease and the like.

When an intestinal disease is prevented or treated or when a symptom thereof is relieved, improvement of the nutrient absorption in the intestinal tract, a decrease in the speed of the intestinal tract transport, an increase in appetite, an increase in the body weight, inhibition of weight loss, prevention of an allergic disease/relief of a symptom thereof, reduction in anxiety state of the brain, improvement of cognitive function/inhibition of its deterioration or the like is expected to be caused.

### <Agent for Improving Lung Function and Composition for Improving Lung Function>

The agent for improving lung function according to the invention contains a bacterium belonging to *Bifidobacterium longum* subsp. *infantis.* The agent for improving lung function according to the invention can improve the lung function of a subject of the intake or the administration.

The composition according to an embodiment of the invention is a composition for improving lung function and contains a bacterium belonging to *Bifidobacterium longum* subsp. *infantis.* The composition for improving lung function according to the invention may contain the agent for improving lung function according to the invention or may be the agent for improving lung function according to the invention itself. The composition for improving lung function according to the invention can improve the lung function of a subject of the intake or the administration.

The lungs are organs playing a role of taking oxygen from the air into the body and expelling carbon dioxide from the body. The air inhaled through the mouth and the nose reaches alveoli at the end of the bronchioles through the pharynx, the trachea and the bronchi. The alveoli are places for gas exchange and supply oxygen to the erythrocytes flowing in the capillary vessels stretching around the alveoli and take carbon dioxide out. In the present specification, the lung function mainly refers to the gas exchange function, and the improvement of lung function specifically includes the forms of an increase in the lung volume, inhibition of a decrease in the lung volume, maintenance of the shape and the stretchability of the alveoli, an increase in the blood oxygen concentration, a decrease in the blood carbon dioxide concentration, improvement of shortness of breath and suffocating, improvement of cough, sputum, and fever, relief of chest pain sensation and pressure, avoidance of respiratory distress and the like.

### <Agent for Preventing or Treating Lung Disease or Relieving Symptom Thereof and Composition for Preventing or Treating Lung Disease or Relieving Symptom Thereof>

The agent for preventing or treating a lung disease or relieving a symptom thereof according to the invention contains a bacterium belonging to *Bifidobacterium longum* subsp. *infantis.* The agent for preventing or treating a lung disease or relieving a symptom thereof according to the invention can prevent or treat a lung disease or relieve a symptom thereof in a subject of the intake or the administration.

The composition according to an embodiment of the invention is a composition for preventing or treating a lung disease or relieving a symptom thereof and contains a bacterium belonging to *Bifidobacterium longum* subsp. *infantis.* The composition for preventing or treating a lung disease or relieving a symptom thereof according to the invention may contain the agent for preventing or treating a lung disease or relieving a symptom thereof according to the invention or may be the agent for preventing or treating a lung disease or relieving a symptom thereof according to the invention itself. The composition for preventing or treating a lung disease or relieving a symptom thereof according to the invention can prevent or treat a lung disease or relieve a symptom thereof in a subject of the intake or the administration. The relief of a symptom includes relief of a symptom accompanying the disease, delay in progress of the disease and reduction in the period required for the recovery of the condition.

In the present specification, the lung disease means a disease in the lungs. The disease of the lungs is not particularly limited, but examples thereof include infectious diseases, interstitial lung disease, emphysema, lung tumor, allergic lung diseases and the like.

An example of the lung disease is pneumonia, and the agent for preventing or treating a lung disease or relieving a symptom thereof and the composition for preventing or treating a lung disease or relieving a symptom thereof according to the invention is believed to be effective especially against pneumonia. Examples of pneumonia include infection with a microorganism such as bacteria and viruses, tuberculosis, lung abscess, mycoses, hypersensitivity pneumonitis, interstitial pneumonia and the like. The viruses which cause pneumonia include rhinoviruses, coronaviruses, MERS-coronaviruses, SARS-coronaviruses, RS virus, influenza viruses, parainfluenza viruses, adenoviruses, human metapneumovirus and the like. The bacteria which cause pneumonia include pneumococci, *Haemophilus influenzae, Mycoplasma pneumoniae, Staphylococcus aureus, Chlamydia* bacteria, *Legionella* bacteria and the like. For example, *Mycoplasma pneumoniae* is a bacterium which easily causes a community-acquired infection, damages the mucosa of the upper respiratory tract, the trachea, the bronchi, the alveoli or the like and causes pneumonia. Infection with *Mycoplasma pneumoniae* easily causes infection with other bacteria, viruses and the like, and the lesion tends to become severe. There are many infections, including infection with *Mycoplasma pneumoniae,* against which no vaccine is available. The composition for preventing or treating a lung disease or relieving a symptom thereof according to the invention can prevent or treat also an infection against which no vaccine is available and which is difficult to prevent or can relieve a symptom thereof.

When a lung disease is prevented or treated or when a symptom thereof is relieved, an increase in appetite, growth of an individual, an increase in the body weight, inhibition of weight loss or the like is expected. Moreover, because a lung disease causes a decrease in the above lung function, prevention or treatment of the lung disease or relief of a symptom thereof can improve the lung function or inhibit a decrease in the lung function.

A lung disease like pneumonia can especially be a lethal infection for infants and small children with insufficient immune function, and thus prevention or treatment of a lung disease or relief of a symptom thereof in infants and small children is important. When a damage of alveoli such as atrophy is caused by a lung disease, the shape of the alveoli cannot be recovered even after the disease is cured. Also in view of this point, prevention or treatment of a lung disease or relief of a symptom thereof in the growth period of the body of infants, small children or the like is important.

### <Agent for Improving Immune Function and Composition for Improving Immune Function>

The agent for improving immune function according to the invention contains a bacterium belonging to *Bifidobacterium longum* subsp. *infantis.* The agent for improving immune function according to the invention can improve the immune function of a subject of the intake or the administration.

The composition for improving immune function according to the invention contains a bacterium belonging to *Bifidobacterium longum* subsp. *infantis.* The composition for improving immune function according to the invention may contain the agent for improving immune function according to the invention or may be the agent for improving immune function according to the invention itself. The composition for improving immune function according to the invention can improve the immune function of a subject of the intake or the administration.

Improvement of immune function can reduce development of an infection, reduce the risk of a malignant tumor or reduce onset of an allergic symptom. Even in these cases, improvement of immune function can relieve a symptom, delay progress of a symptom or reduce the period required for the recovery of the condition.

In the present specification, the immune function includes natural immunity and acquired immunity. The improvement of immune function specifically includes the forms of activation and adequate inhibition of immune cells (including macrophages, dendritic cells, neutrophils, T cells, B cells and NK cells), regulation of differentiation of immune cells, regulation of cytokine production (including promotion and inhibition of production), inhibition of a viral infection, inhibition of a bacterial infection, inhibition of a fungal infection, parasite removal, promotion of antibody production and the like. The cytokines include inflammatory cytokines and anti-inflammatory cytokines.

Phagocytes such as macrophages, dendritic cells and neutrophils devour and degrade pathogens and the like. NK cells release cytotoxic factors and destroy host cells infected with a pathogen. B cells produce antibodies and conduct inactivation of pathogens and the like. Cytokines play an important role in activation and inhibition of such immune cells. Cytokines include chemokines, interferons, interleukins, tumor necrosis factor and the like. Helper T cells can be classified into Th1 cells, Th2 cells and Th17 cells. Th1 cells involve in cell-mediated immunity, release cytokines including IFN-γ and activate NK cells and macrophages. Th2 cells involve in humoral immunity, release cytokines including IL-4 and promote antibody production.

Chemokines are secretory proteins which cause migration of leukocytes and the like and which involve in formation of inflammation, and chemokines are classified into four subfamilies of CC, CXC, C and CX3C based on the structure. Monocyte chemotactic protein 1 (MCP1) belonging to the CC subfamily is expressed in monocytes, macrophages and endothelial cells . It has been reported that MCP1 is increased in the serum of patients with acute myocarditis and is up-regulated in many types of central nervous system (CNS) disorder including ischemia, hemorrhage, injury, an infection, hypoxia and peripheral nerve injury. It has been suggested that MCP1 also involves in diseases characterized by monocyte infiltration such as psoriasis, chronic rheumatoid arthritis and atherosclerosis.

Interleukin-6 (IL-6) is produced from various cells including T cells and macrophages. IL-6 plays an important role in inflammatory response, and for example, IL-6 facilitates production of chemokines such as IL-8 and MCP-1, facilitates expression of cell adhesion molecules such as ICAM-1 and VCAM-1, promotes differentiation of B cells into antibody-producing cells and inhibits regulatory T cells. Interleukin-8 (IL-8) is produced from macrophages, epithelial cells, vascular endothelial cells and the like. IL-8 induces migration of neutrophils to the infected site and induces phagocytosis.

Interferons are secreted by cells in response to entry of a pathogen such as viruses. As interferons, type I interferons, type II interferons and type III interferons are known. Interferons-α and β (IFN-α and β), which are type I interferons, are produced in many cells such as T cells, B cells, macrophages, fibroblasts and vascular endothelial cells and are especially important for antiviral response. IFN-α and β activate macrophages and NK cells. Interferon-γ (IFN-γ), which is a type II interferon, is secreted from T cells, NK cells and the like. IFN-γ enhances inflammation and promotes the phagocytosis of macrophages, dendritic cells and the like.

The composition for improving immune function or the agent for improving immune function according to the invention can regulate production of a cytokine including at least one selected from the group consisting of interleukin-6, interleukin-8, monocyte chemotactic protein, interferon-β and interferon-γ. The composition or the agent according to the invention can promote production of an inflammatory cytokine. The composition or the agent according to the invention can promote production of a cytokine including at least one selected from the group consisting of interleukin-6, interleukin-8, monocyte chemotactic protein, interferon-β and interferon-γ.

The composition for improving immune function or the agent for improving immune function according to the invention can inhibit infection with a pathogenic microorganism such as a virus, a bacterium and a fungus. Cells infected with a pathogenic microorganism are eliminated by killer T cells, NK cells and the like. CD8 is a marker of killer T cells and can be used as an indicator for infection with a pathogenic microorganism. It can be determined that infection with pathogenic microorganisms is high when the expression level of CD8 is high and that infection with pathogenic microorganisms is inhibited when the expression level of CD8 is low.

Promotion of production of the above cytokines improves immune function not only in the intestinal tract but also in the whole body. As a result, infection with a pathogenic microorganism can be prevented or treated, or a symptom thereof can be relieved, in various sites such as the whole body, central nervous system, respiratory organs, digestive organs, urinary tract, skin and lymph nodes.

Because the immune function of infants and small children is not developed sufficiently, improvement of immune function is especially important for infants and small children.

### <Bacterium Belonging to Bifidobacterium longum subsp. infantis>

A bacterium belonging to *Bifidobacterium longum* subsp. *infantis* is a Gram-positive obligately anaerobic bacillus. As the bacterium belonging to *Bifidobacterium longum* subsp. *infantis,* any one, two or more kinds of known or unknown bacteria belonging to *Bifidobacterium longum* subsp. *infantis* can be selected as long as the effects of the invention are not impaired. *Bifidobacterium longum* subsp. *infantis* is sometimes simply called with an abbreviation of *Bifidobacterium infantis.* The bacterium belonging to *Bifidobacterium longum* subsp. *infantis* is *Bifidobacterium longum* subsp. *infantis* BCCM LMG23728, *Bifidobacterium longum* subsp. *infantis* M-63 (NITE BP-02623) or the like.

*Bifidobacterium longum* subsp. *infantis* BCCM LMG23728 can be acquired from Belgian Coordinated Collections of Microorganisms (BCCM) (address: Rue de la Science (Wetenschapsstraat) 8, Brussels B-1000, Belgium), which is a depositary authority in Belgium.

*Bifidobacterium longum* subsp. *infantis* M-63 (NITE BP-02623) was deposited for an international deposit under the Budapest Treaty on January 26, 2018 to NITE Patent Microorganisms Depositary, National Institute of Technology and Evaluation (Room 122, 2-5-8 Kazusakamatari, Kisarazu-shi, Chiba 292-0818) and given the accession number of NITE BP-02623. *Bifidobacterium longum* subsp. *infantis* BCCM LMG23728 and *Bifidobacterium longum* subsp. *infantis* M-63 (NITE BP-02623) are substantially identical.

The bacterial shape of *Bifidobacterium longum* subsp. *infantis* M-63 is a bacillus or branched polymorphic, and the bacterium does not form spores and is not motile. When the bacterium is spread on a BL agar plate and cultured in an anaerobic condition at 37°C for 48 hours, an opaque, circular hemispheric, shining colony is formed. *Bifidobacterium longum* subsp. *infantis* M-63 is fermentative for arabinose, xylose, glucose, fructose, galactose, sucrose, maltose, lactose, melibiose, raffinose and turanose.

As the bacterium belonging to *Bifidobacterium longum* subsp. *infantis* contained in the composition or the agent according to the invention, a bacterium which is substantially identical to the deposited strain may be contained. Examples of the bacterium which is substantially identical to the deposited strain include strains derived from the deposited strain as the parent strain. The derived strains include strains bred from the deposited strain and strains naturally generated from the deposited strain.

In the present specification, "the substantially identical bacterium" is a bacterium of the same genus or the same species and is a bacterium in which the nucleotide sequence of 16S rRNA gene has an identity of 98% or more, preferably 99% or more, more preferably 100% to the nucleotide sequence of 16S rRNA gene of the bacterium and which preferably has the identical bacterial features to those of the bacterium. The substantially identical bacterium may be (1) a bacterium which is determined to be the identical strain by the RAPD (Randomly Amplified Polymorphic DNA) method or the PFGE (Pulsed-field gel electrophoresis) method (described in Probiotics in food/Health and nutritional properties and guidelines for evaluation 85 Page43), (2) a bacterium which has the genes derived from the bacterial strain only but does not have any exogenous gene and which has a DNA identity of 95% or more or (3) a bacterium of a strain bred from the bacterial strain (including modification by genetic engineering, a mutation or a spontaneous mutation and having the identical characters).

The bacterium belonging to *Bifidobacterium longum* subsp. *infantis* contained in the composition or the agent according to the invention may be a living bacterium or a dead bacterium or may be both a living bacterium and a dead bacterium, but the bacterium is preferably a living bacterium. A microorganism which is taken or administered alive and which provides beneficial effects on the host is also called a probiotic. The dead bacterium may be a homogenate. As long as the effects of the invention are not impaired, an additional operation such as heating or lyophilization may be conducted after culturing. The additional operation is preferably an operation in which the survival rate of the living bacterium is high.

The bacterium belonging to *Bifidobacterium longum* subsp. *infantis* contained in the composition or the agent according to the invention can be cultured by a method which is generally used for culturing *Bifidobacterium* bacteria (bifidobacteria) with appropriate modification. The culture temperature may be, for example, 25 to 50°C and is preferably 30 to 40°C. The bacterium is preferably cultured under an anaerobic condition and can be cultured, for example, while sending an anaerobic gas such as carbon dioxide gas. The pH of the medium is not particularly limited as long as the pH is in the range in which the bacterium can grow, but the pH is, for example, pH6.0 to 8.0.

The medium for culturing the bacterium belonging to *Bifidobacterium longum* subsp. *infantis* contained in the composition or the agent according to the invention is not particularly limited, and a medium which is generally used for culturing the bacterium can be used with appropriate modification when necessary. As the carbon source of the medium, for example, saccharides such as galactose, glucose, fructose, mannose, cellobiose, maltose, lactose, sucrose, trehalose, starch, starch hydrolysate and molasses can be used depending on the assimilation properties. As the nitrogen source of the medium, for example, ammonia, ammonium salts and nitrates such as ammonium sulfate, ammonium chloride and ammonium nitrate and the like can be used. As the inorganic salt of the medium, for example, sodium chloride, potassium chloride, potassium phosphate, magnesium sulfate, calcium chloride, calcium nitrate, manganese chloride, ferrous sulfate and the like can be used. Organic components such as peptone, soybean powder, a defatted soybean cake, meat extract and yeast extract may also be used for the medium. As a modified medium, for example, MRS medium can be preferably used.

The bacterium belonging to *Bifidobacterium longum* subsp. *infantis* can be used for promotion of development of the intestinal tract, prevention or treatment of an intestinal disease, relief of a symptom thereof, improvement of lung function, prevention or treatment of a lung disease, relief of a symptom thereof or improvement of immune function. Because the bacterium belonging to *Bifidobacterium longum* subsp. *infantis* has been long used in foods, drugs and the like and exists in the intestines of animals as a good bacterium, the bacterium is expected to be highly safe for the living body. Moreover, because the possibility of side-effects and dependency is believed to be low, the bacterium can be taken continuously for a long period and can be taken by the subject of application easily.

The composition or the agent according to the invention may contain a bacterium other than the bacterium belonging to *Bifidobacterium longum* subsp. *infantis* as long as the effect of promoting development of the intestinal tract, the effect of preventing or treating an intestinal disease or relieving a symptom thereof, the effect of improving lung function, the effect of preventing or treating a lung disease or relieving a symptom thereof and the effect of improving immune function are not impaired. Examples of the bacterium which can be contained in the composition or the agent according to the invention include other *Bifidobacterium* bacteria and lactic acid bacteria. Examples of the other *Bifidobacterium* bacteria include *Bifidobacterium bifidum, Bifidobacterium longum* subsp. *longum, Bifidobacterium adolescentis, Bifidobacterium pseudocatenulatum, Bifidobacterium animalis* subsp. *lactis, Bifidobacterium breve, Bifidobacterium pseudolongum* and the like. The lactic acid bacteria include *Lactobacillus paracasei, Lactobacillus plantarum, Lactobacillus acidophilus, Lactobacillus rhamnosus, Lactobacillus gasseri* and the like.

The composition or the agent according to the invention may contain a culture of the bacterium belonging to *Bifidobacterium longum* subsp. *infantis.* The culture may be diluted or concentrated. The culture of the bacterium can contain secreted materials and metabolites of the bacterium.

### <Food or Drink Composition>

The composition according to the invention can be widely used as a medicine, a food or a drink and feed. The composition is preferably taken orally or through a tube and is more preferably orally taken. The composition can be taken daily in the form of a medicine or a food or a drink. The composition of the invention is preferably a food or drink composition. The food or drink composition according to the invention is safe and can easily promote development of the intestinal tract, prevent or treat an intestinal disease, relieve a symptom thereof, promote improvement of lung function, prevent or treat a lung disease, relieve a symptom thereof or improve immune function.

The food or drink composition according to the invention is not limited regarding the form such as liquid, paste, solid and powder and also includes the form of tablet candies, liquid foods or the like. Examples of the food or drink composition according to the invention include wheat products, instant foods, processed agricultural products, processed fishery products, processed livestock products, milk/dairy products, oils and fats, basic condiments, compound flavor enhancers/foods, frozen foods, confectioneries, beverages, other commercial products and the like.

Examples of the wheat products include breads, macaroni, spaghetti, noodles, cake mixes, frying flours, bread crumbs and the like. Examples of the instant foods include instant noodles, cup noodles, retort-pouched/prepared foods, prepared canned foods, microwave foods, instant soups/stews, instant miso soups/clear Japanese soups, canned soups, freeze-dried foods, other instant foods and the like. Examples of the processed agricultural products include canned agricultural products, canned fruits, jams/marmalades, pickles, cooked beans, dried agricultural products, cereals (processed grains) and the like. Examples of the processed fishery products include canned fishery products, fish hams/sausages, fishery paste products, fishery delicacies, *Tsukudani* (foods boiled down in sweetened soy sauce) and the like. Examples of the processed livestock products include canned livestock products/pastes, livestock hams/sausages and the like. Examples of the milk/dairy products include fermented milk, processed milk, milk beverages, yogurts, lactic acid bacteria beverages, cheeses, ice creams, modified powder milk, creams, other dairy products and the like. Examples of the oils and fats include butter, margarine, vegetable oils and the like. Examples of the basic condiments include soy sauce, soybean paste, sauces, processed tomato condiments, *Mirin* (sweet sake for seasoning), vinegars and the like. The compound flavor enhancers/foods include cooking mixes, curry roux, sauces, dressings, noodle broths, spices, other compound flavor enhancers and the like. Examples of the frozen foods include frozen food materials, semi-cooked frozen foods, cooked frozen foods and the like. Examples of the confectioneries include caramels, candies, chewing gums, chocolates, cookies, biscuits, cakes, pies, snacks, crackers, Japanese-style confectioneries, rice confectioneries, bean confectioneries, desserts, other confectioneries and the like. Examples of the beverages include carbonated drinks, natural juices, fruit juices, fruit juice-containing soft drinks, fruit flesh drinks, fruit granule-containing fruit juices, vegetable drinks, soy milk, soy milk drinks, coffee drinks, tea drinks, drink powders, concentrated drinks, sport drinks, nutritional drinks, alcohols, other luxury beverages and the like. Examples of the other commercial foods include baby foods, *Furikake* (dry Japanese seasonings), seasonings for *Chazuke* (boiled rice with hot tea) and the like.

The amount of the bacterium belonging to *Bifidobacterium longum* subsp. *infantis* in the food or drink composition is not particularly limited and can be appropriately selected based on the daily intake. The amount of the bacterium belonging to *Bifidobacterium longum* subsp. *infantis* in the food or drink composition is, for example, preferably 1×10⁶ to 1×10¹² cfu/g or 1×10⁶ to 1×10¹² cfu/mL, more preferably 1×10⁷ to 1×10¹¹ cfu/g or 1×10⁷ to 1×10¹¹ cfu/mL, further preferably 1×10⁸ to 1×10¹⁰ cfu/g or 1×10⁸ to 1×10¹⁰ cfu/mL. The unit cfu is an abbreviation of colony forming units and is the colony forming unit. When the bacterium is a dead bacterium, cfu/g or cfu/mL can be replaced with cells/g or cells/mL.

The amount of the bacterium belonging to *Bifidobacterium longum* subsp. *infantis* in the food or drink composition can be set at any amount as long as the effects of the invention are not impaired. The amount of the bacterium belonging to *Bifidobacterium longum* subsp. *infantis* in the food or drink composition can be set at 0.3 mass% to 5.0 mass% relative to the final composition of the food or drink composition.

The intake of the food or drink composition, as the amount of the bacterium belonging to *Bifidobacterium longum* subsp. *infantis* per 1 kg body weight of the subject of the administration or the intake, is preferably 1×10⁶ to 1×10¹² cfu/day, more preferably 1×10⁷ to 1×10¹¹ cfu/day, further preferably 1×10⁸ to 1×10¹⁰ cfu/day. When the bacterium is a dead bacterium, cfu/day can be replaced with cells/day.

The food or drink composition may be a modified powder milk or a modified liquid milk. The modified powder milk is an infant modified powder milk or modified liquid milk, a follow-up modified powder milk or modified liquid milk, a modified powder milk or a modified liquid milk for low birthweight infants, a modified powder milk or a modified liquid milk for small children, a modified powder milk or a modified liquid milk for adults, a modified powder milk or a modified liquid milk for the aged, a modified powder milk or a modified liquid milk for allergies, a modified powder milk or a modified liquid milk for lactose intolerance, a modified powder milk or a modified liquid milk for inborn error of metabolism or the like. The food or drink composition is especially preferably an infant modified powder milk or modified liquid milk, a follow-up modified powder milk or modified liquid milk, a modified powder milk or a modified liquid milk for low birthweight infants or a modified powder milk or a modified liquid milk for small children.

The composition can contain various additives depending on the kind thereof. The composition preferably contains a human milk oligosaccharide. Human milk oligosaccharide is a generic term for oligosaccharides of tri- and higher saccharides contained in human milk excluding lactose. Mammalian milk has core skeletons of 12 series such as lacto-N-tetraose (LNT), lacto-N-neotetraose (LNnT), lacto-N-hexaose (LNH) and lacto-N-neohexaose (LNnH) . Addition of Lewis a, b or x or a2-3/2-6 N-acetylneuraminic acid to the skeletons results in 100 variations of sugar chains. Human milk oligosaccharides are contained in the breast milk in an amount of 10 to 20 g/L, and 130 kinds of oligosaccharides or more can be contained.

As the human milk oligosaccharide contained in the composition, any one, two or more kinds of known or unknown human milk oligosaccharides can be selected and used as long as the bacterium belonging to *Bifidobacterium longum* subsp. *infantis* can use the oligosaccharides and as long as the effects of the invention are not impaired. Examples of the human milk oligosaccharide include L-fucose, Lacto-N-fucopentaose I, Lacto-N-fucopentaose II, 2'-Fucosyllactose, 3'-Fucosyllactose, Lacto-N-fucopentaose III, 3'-Fucosyllacto-N-hexaose, Lacto-N-difucohexaose I, Lacto-difucotetraose, Lacto-difucohexaose I, Lactodifucohexaose II, Lacto-N-tetraose, Difucosyl syalyllacto-N-hexaose, Syalyllacto-N-tetraose, Fucosylsyalyl lacto-N-hexaose I, 3'-Fucosylsialyl lacto-N-tetraose (Fuc 1,2 Gal, Fuc 1,4 Gal NAC), Lacto-N-neotetraose, 3'-Sialyl-3-fucosyllactose, Disialomonofucosyllacto-N-neohexaose, Monofucosylmonosialyl lacto-N-octaose, Sialyllacto-N-fucohexaose II, Disialyllacto-N-fucopentaose II, Monofucosyldisialyllacto-N-tetraose, 2'-Sialyllactose, 2'-Sialyllactosamine, 3'-Sialyllactose, 3'-Sialyllactosamine, 6'-Sialyllactose, 6'-Sialyllactosamine, Sialyllacto-N-neotetraose c, Monosialyllacto-N-hexaose, Disialyllacto-N-hexaose I, Monosialyllacto-N-neohexaose I, Monosialyllacto-N-neohexaose II, Disialyllacto-N-neohexaose, Disialyllacto-N-tetraose, Disialyllacto-N-hexaose II, Sialyllacto-N-tetraose a, Disialyllacto-N-hexaose I, Sialyllacto-N-tetraose b, 2'-Fucosyllacto-N-hexaose, 3'-Fucosyllacto-N-neohexaose, 2'-Fucosyl-N-acetylglucosamine, Lacto-N-fucopentaose V, Lacto-N-hexaose, Para-lacto-N-hexaose, Lacto-N-neohexaose, Para-lacto-N-neohexaose, Monofucosyllacto-N-hexaose II, Isomeric fucosylated lacto-N-hexaose (1), Isomeric fucosylated lacto-N-hexaose (3), Isomeric fucosylated lacto-N-hexaose (2), Difucosyl-para-lacto-N-neohexaose, Difucosyl-para-lacto-N-hexaose, 2' 3'-Difucosyllacto-N-hexaose, Lacto-N-neoocataose, Para-lacto-N-octanose, Iso-lacto-N-octaose, Lacto-N-octaose, Monofucosyllacto-neoocataose, Monofucosyllacto-N-ocataose, Difucosyllacto-N-octaose I, Difucosyllacto-N-octaose II, Difucosyllacto-N-neoocataose II, Difucosyllacto-N-neoocataose I, Lacto-N-decaose, Trifucosyllacto-N-neooctaose, Trifucosyllacto-N-octaose, Trifucosyl-iso-lacto-N-octaose, Lacto-N-difuco-hexaose II, Sialyl-lacto-N-tetraose a, Sialyl-lacto-N-tetraose b, Sialyl-lacto-N-tetraosec, Sialyl-fucosyl-lacto-N-tetraoseI, Sialyl-fucosyl-lacto-N-tetraose II, Disialyl-lacto-N-tetraose, Lacto-N-neotetraose and the like and combinations thereof. The composition of the invention preferably contains at least one selected from 2'-fucosyllactose (also called "2'-FL" below), lacto-N-fucopentaose I (also called "LNFPI" below), lacto -N-difucohexaose I (also called "LNDFP I" below) and lacto-N-tetraose (also called "LNnT" below).

2'-FL and LNnT are known to have the bifidobacterium-growing activity and act as a growth factor for bifidobacteria by being selectively used by bifidobacteria and are important for the formation of enteric microbiota in which bifidobacteria are dominant. The human milk oligosaccharide can be synthesized and used industrially.

The method for adding the human milk oligosaccharide is not particularly limited as long as the effects of the invention are not impaired, but for example, the composition of the invention can be produced by adding and mixing the human milk oligosaccharide after formation into liquid or powder.

The human milk oligosaccharide content can be set at any content as long as the effects of the invention are not impaired. In the invention, the human milk oligosaccharide content of the composition can be set at 0.3 mass% to 5.0 mass% relative to the final composition of the composition.

The composition may further contain a prebiotic. The prebiotic is a resistant food component which has an advantageous effect on the host and improves the health of the host by selectively changing the growth and the activities of specific bacteria in the large intestine. The prebiotic is not particularly restricted as long as the effects of the invention are not impaired but is preferably, for example, lactulose, raffinose, a galactooligosaccharide, a fructooligosaccharide, a soybean oligosaccharide, lactosucrose, a xylooligosaccharide, an isomaltooligosaccharide, a coffee bean mannooligosaccharide, gluconic acid, polydextrose, inulin or the like. Of these, lactulose, raffinose, a galactooligosaccharide and the like are more preferable, and lactulose, raffinose and a galactooligosaccharide are further preferable.

The food or drink composition of the invention can be produced by a method known to one skilled in the art. The food or drink composition may be produced by adding the bacterium belonging to *Bifidobacterium longum* subsp. *infantis* to a known food or a known drink, or a new food or drink composition can be produced by mixing the bacterium into materials of a food or a drink. The food composition can be produced by an appropriate combination of a forming step, a sterilization step, a fermentation step, a baking step, a drying step, a cooling step, a granulation step, a packaging step and the like as well as by a step of adding and mixing the bacterium belonging to *Bifidobacterium longum* subsp. *infantis.* The bacterium belonging to *Bifidobacterium longum* subsp. *infantis* may also be used as a starter for producing fermented milk.

The amount of the bacterium belonging to *Bifidobacterium longum* subsp. *infantis* in the production of the composition is not particularly limited but is preferably, for example, 1×10⁶ to 1×10¹² cfu/g or 1×10⁶ to 1×10¹² cfu/mL, more preferably 1×10⁷ to 1×10¹¹ cfu/g or 1×10⁷ to 1×10¹¹ cfu/mL, further preferably 1×10⁸ to 1×10¹⁰ cfu/g or 1×10⁸ to 1×10¹⁰ cfu/mL. When the bacterium is a dead bacterium, cfu/g or cfu/mL can be replaced with cells/g or cells/mL.

The food or drink composition for promoting development of the intestinal tract and the food or drink composition for preventing or treating an intestinal disease or relieving a symptom thereof can be provided or sold as a food or a drink with a label with a health use such as development of the intestinal tract, prevention or treatment of an intestinal disease and a relief of a symptom thereof. Such a label is not particularly limited, but examples thereof include "promote development of the intestinal tract", "improve the ability of absorbing nutrients", "help digestion", "increase the body weight", "promote growth", "inhibit intestinal inflammation", "prevent an intestinal disease", "prevent and treat an infection", "prevent and treat food poisoning", "prevent and treat food allergy", "prevent and treat atopic dermatitis", "prevent and treat allergic rhinitis", "prevent and treat asthma", "prevent and treat anxiety symptoms" and "improve cognitive function/inhibit its deterioration". The "labeling" act includes all the acts for informing a consumer of the use, and all the expressions which can remind of/cause to guess the use are the "labeling" acts of the invention, regardless of the purposes of the labels, the contents of the labels, the objects to be labeled, the media and the like.

The food or drink composition for improving lung function and the food or drink composition for preventing or treating a lung disease or relieving a symptom thereof can be provided or sold as a food or a drink with a label with a health use such as improvement of lung function, prevention or treatment of a lung disease and relief of a symptom thereof. Such a label is not particularly limited, but examples thereof include "improve lung function", "help development of the lungs", "promote growth", "prevent a lung disease", "prevent pneumonia", "inhibit lung inflammation", "maintain health of the lungs" and "protect the lungs from an infection".

The food or drink composition for improving immune function can be provided or sold as a food or a drink with a label with a health use such as improvement of immune function. Such a label is not particularly limited, but examples thereof include "improve immune function", "improve immunocompetence", "prevent common cold", "promote health", "inhibit a viral infection", "inhibit a bacterial infection", "inhibit a fungal infection", "remove a parasite", "fatigue-resistant" and "relieve chilliness".

The content of the label is preferably a label approved by the administration or the like (for example, a label approved based on a system provided by the administration and provided in the form based on the approval or the like) . It is preferable to label with such a content on packaging, a container, a catalogue, a brochure, an advertisement material in a sales site such as POP, other documents or the like.

The "labels" also include labels with health foods, functional foods, enteral nutrition products, food for special dietary uses, food with health claims, foods for specified health uses, foods with nutrient function claims, foods with function claims, quasi-drugs and the like. In particular, the labels are labels approved by the Consumer Affairs Agency, such as labels approved by the systems for foods for specified health uses, foods with nutrient function claims or foods with function claims and labels approved by a similar system. Specific examples include a label with foods for specified health uses, a label with qualified foods for specified health uses, a label with foods with function claims, a label indicating influence on the structure or the function of a body, a label with reduction of disease risk, a label with a scientifically grounded function and the like.

### <Pharmaceutical Composition>

The pharmaceutical composition is not particularly restricted as long as the bacterium belonging to *Bifidobacterium longum* subsp. *infantis* is contained. For the pharmaceutical composition, the bacterium belonging to *Bifidobacterium longum* subsp. *infantis* may be directly used, or the pharmaceutical composition may be produced by blending a physiologically acceptable liquid or a carrier for formulation.

The dosage form of the pharmaceutical composition is not particularly restricted, and the pharmaceutical composition can be formulated into a solid preparation such as powder, granules, tablets and capsules, a liquid preparation such as a solution, a syrup, a suspension and an emulsion, a suppository, ointment or the like. For the formulation, a carrier for formulation which is used for general formulation can be used. The pharmaceutical composition can also contain a component which is known or will be found in the future and which has the activity of promoting development of the intestinal tract, the activity of preventing or treating an intestinal disease or relieving a symptom thereof, the activity of improving lung function, the activity of preventing or treating a lung disease or relieving a symptom thereof or the activity of improving immune function.

The amount of the bacterium belonging to *Bifidobacterium longum* subsp. *infantis* in the pharmaceutical composition is not particularly limited and can be appropriately selected based on the daily intake or dose corresponding to the dosage form. The amount of the bacterium belonging to *Bifidobacterium longum* subsp. *infantis* in the pharmaceutical composition is preferably, for example, 1×10⁶ to 1×10¹² cfu/g or 1×10⁶ to 1×10¹² cfu/mL, more preferably 1×10⁷ to 1×10¹¹ cfu/g or 1×10⁷ to 1×10¹¹ cfu/mL, further preferably 1×10⁸ to 1×10¹⁰ cfu/g or 1×10⁸ to 1×10¹⁰ cfu/mL. When the bacterium is a dead bacterium, cfu/g or cfu/mL can be replaced with cells/g or cells/mL.

The dosage of the pharmaceutical composition, as the amount of the bacterium belonging to *Bifidobacterium longum* subsp. *infantis* per 1 kg body weight of the subject of the administration, is preferably 1×10⁶ to 1×10¹² cfu/day, more preferably 1×10⁷ to 1×10¹¹ cfu/day, further preferably 1×10⁸ to 1×10¹⁰ cfu/day. When the bacterium is a dead bacterium, cfu/day can be replaced with cells/day. The administration method of the pharmaceutical composition may be oral administration or parenteral administration. The pharmaceutical composition may be administered once a day or in divided portions.

As the carrier for formulation, an organic or inorganic carrier can be used depending on the dosage form. Examples of the carrier for a solid preparation include excipients, binders, disintegrating agents, lubricants, stabilizers, flavoring agents and the like.

Examples of the excipients include: saccharide derivatives such as lactose, sucrose, glucose, mannitol and sorbitol; starch derivatives such as cornstarch, potato starch, α-starch, dextrin and carboxymethyl starch; cellulose derivatives such as crystalline cellulose, hydroxypropyl cellulose, hydroxypropyl methylcellulose, carboxymethylcellulose and carboxymethyl cellulose calcium; gum arabic; dextran; pullulan; silicate derivatives such as light silicic anhydride, synthetic aluminum silicate and magnesium aluminometasilicate; phosphate derivatives such as calcium phosphate; carbonate derivatives such as calcium carbonate; sulfate derivatives such as calcium sulfate; and the like.

Examples of the binders include, in addition to the excipients, gelatin, polyvinylpyrrolidone and macrogol.

Examples of the disintegrating agents include, in addition to the excipients, chemically modified starch or cellulose derivatives such as croscarmellose sodium, sodium carboxymethyl starch and cross-linked polyvinylpyrrolidone and the like.

Examples of the lubricants include: talc; stearic acid; metal stearates such as calcium stearate and magnesium stearate; colloidal silica; waxes such as pea gum and spermaceti wax; boric acid; glycols; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acid such as silicic anhydride and silicic acid hydrate; starch derivatives; and the like.

Examples of the stabilizers include: paraoxybenzoate esters such as methylparaben and propylparaben; alcohols such as chlorobutanol, benzyl alcohol and phenylethyl alcohol; benzalkonium chloride; acetic anhydride; sorbic acid; and the like.

Examples of the flavoring agents include sweeteners, acidulants, aromas and the like. In this regard, the carriers used in the case of a liquid preparation for oral administration include solvents such as water, flavoring agents and the like.

The pharmaceutical composition may be administered alone or used in combination with another medicine, such as another medicine for promoting development of the intestinal tract, a medicine for preventing or treating an intestinal disease or relieving a symptom thereof, an intestinal regulator, a gastrointestinal agent, another medicine for an intestinal disease, a medicine for improving lung function, a medicine for preventing or treating a lung disease or relieving a symptom thereof, a medicine for improving immune function, an antibiotic, a virus inhibitor, an anti-inflammatory agent and an analgesic agent.

### <Feed Composition>

Examples of the feed composition according to the invention include pet food, livestock feed, fish farming feed and the like. The feed composition can be produced by mixing the bacterium belonging to *Bifidobacterium longum* subsp. *infantis* to general feed or materials thereof such as grains, lees, bran, fish meal, bone meal, oils and fats, defatted milk powder, whey, mineral feed and yeasts. For example, the feed composition may be produced through a fermentation step by the added bacterium belonging to *Bifidobacterium longum* subsp. *infantis* like silage. The produced feed composition can be orally administered to general mammals, domestic animals, farmed fish, pets and the like.

The amount of the bacterium belonging to *Bifidobacterium longum* subsp. *infantis* in the feed composition is appropriately set depending on the form of the feed composition and the subject of the administration but is preferably, for example, 1×10⁶ to 1×10¹² cfu/g or 1×10⁶ to 1×10¹² cfu/mL, more preferably 1×10⁷ to 1×10¹¹ cfu/g or 1×10⁷ to 1×10¹¹ cfu/mL, further preferably 1×10⁸ to 1×10^{1o} cfu/g or 1×10⁸ to 1×10^{1o} cfu/mL. When the bacterium is a dead bacterium, cfu/g or cfu/mL can be replaced with cells/g or cells/mL.

### <Method for Promoting Development of Intestinal Tract or for Preventing or Treating Intestinal Disease or Relieving Symptom Thereof>

The method for promoting development of the intestinal tract or for preventing or treating an intestinal disease or relieving a symptom thereof includes a step of administering a bacterium belonging to *Bifidobacterium longum* subsp. *infantis* to a mammal. The bacterium belonging to *Bifidobacterium longum* subsp. *infantis* may be administered in the form of the above composition containing the bacterium belonging to *Bifidobacterium longum* subsp. *infantis.*

The mammal is human, cattle, sheep, goat, pig, dog, cat, horse or the like. The subject of the administration may be an infant or a small child and may be a human infant or small child. The term infant or small child includes an infant and a small child, further specifically includes an infant, a small child and a newborn and further specifically includes an infant, a small child, a newborn, an immature infant, a premature infant and a low birthweight infant. The administration may be continued also after the infancy or the early childhood. In an infant or a small child, through administration of the bacterium belonging to *Bifidobacterium longum* subsp. *infantis,* the bacterium in the intestines grows easily, and the effect of promoting development of the intestinal tract and the effect of preventing or treating an intestinal disease or relieving a symptom thereof are easily obtained.

### <Method for Improving Lung Function or for Preventing or Treating Lung Disease or Relieving Symptom Thereof>

The method for improving lung function or for preventing or treating a lung disease or relieving a symptom thereof includes a step of administering a bacterium belonging to *Bifidobacterium longum* subsp. *infantis* to a mammal. The bacterium belonging to *Bifidobacterium longum* subsp. *infantis* may be administered in the form of the above composition containing the bacterium belonging to *Bifidobacterium longum* subsp. *infantis.*

In an infant or a small child, through administration of the bacterium belonging to *Bifidobacterium longum* subsp. *infantis,* the bacterium in the intestines grows easily, and the effect of improving lung function and the effect of preventing or treating a lung disease or relieving a symptom thereof are easily obtained.

### <Method for Improving Immune Function>

The method for improving immune function includes a step of administering a bacterium belonging to *Bifidobacterium longum* subsp. *infantis* to a mammal. The bacterium belonging to *Bifidobacterium longum* subsp. *infantis* may be administered in the form of the above composition containing the bacterium belonging to *Bifidobacterium longum* subsp. *infantis.*

In an infant or a small child, through administration of the bacterium belonging to *Bifidobacterium longum* subsp. *infantis,* the bacterium in the intestines grows easily, and the effect of improving immune function is easily obtained.

In the present specification, the infant refers to a child in the infancy, and the infancy refers to the period from birth to before the age of 1 year (until the day before the day on which the subject turns 1 year old) when the subject is a human. The infancy refers to the period in which the subject takes milk such as breast milk as the major source of nutrient.

In the present specification, the small child refers to a child in the early childhood, and the early childhood refers to the period from the day on which the subject turns 1 year old to before entering the school age when the subject is a human. The early childhood preferably refers to the period from the day on which the subject turns 1 year old to the day before earlier one of the day on which the subject turns 6 years old and the first day of elementary school.

When the subject is a human, the composition or the agent according to the invention is preferably taken or administered between birth and before entering the school age. When the subject is a human, the composition or the agent according to the invention is more preferably taken or administered between birth and before the age of 3 years (until the day before the day on which the subject turns 3 years old), further preferably taken or administered in the infancy, still further preferably taken or administered between the age of three months and before the age of 1 year (until the day before the day on which the subject turns 1 year old).

When the subj ect is a mammal other than human, the periods are converted to the corresponding periods.

The "infancy" used when the subject is a human corresponds to the period from birth to the age of 20 days in rats. Similarly, in cattle, the period corresponds to the period from birth to the day before the day on which the subject turns 10 weeks old. In goats, the period corresponds to the period from birth to the day before the day on which the subj ect turns 10 weeks old. In sheep, the period corresponds to the period from birth to the day before the day on which the subj ect turns 12 weeks old. In pigs, the period corresponds to the period from birth to the day before the day on which the subj ect turns 8 weeks old. In monkeys, the period corresponds to the period from birth to the day before the day on which the subj ect turns 14 weeks old. In dogs, the period corresponds to the period from birth to the day before the day on which the subject turns 6 weeks old. In cats, the period corresponds to the period from birth to the day before the day on which the subject turns 6 weeks old. In mice, the period corresponds to the period from birth to the age of 20 days. In hamsters, the period corresponds to the period from birth to the age of 20 days. In guinea pigs, the period corresponds to the period from birth to the age of 20 days.

The "early childhood" used when the subject is a human corresponds to the period from the age of 21 days to the day before the day on which the subject turns 5 weeks old in rats. Similarly, in cattle, the period corresponds to the period from the age of 10 weeks to the day before the day on which the subj ect turns 1 year old. In goats, the period corresponds to the period from the age of 10 weeks to the day before the day on which the subject turns 1 year old. In sheep, the period corresponds to the period from the age of 12 weeks to the day before the day on which the subject turns 1 year old. In pigs, the period corresponds to the period from the age of 8 weeks to the day before the day on which the subject turns 4 months old. In monkeys, the period corresponds to the period from the age of 14 weeks to the day before the day on which the subj ect turns 1.5 years old. In dogs, the period corresponds to the period from the age of 6 weeks to the day before the day on which the subject turns 6 months old. In cats, the period corresponds to the period from the age of 6 weeks to the day before the day on which the subject turns 6 months old. In mice, the period corresponds to the period from the age of 21 days to the day before the day on which the subject turns 5 weeks old. In hamsters, the period corresponds to the period from the age of 21 days to the day before the day on which the subject turns 5 weeks old. In guinea pigs, the period corresponds to the period from the age of 21 days to the day before the day on which the subject turns 5 weeks old.

The "3 years old" used when the subject is a human corresponds to 4 weeks old in rats. Similarly, in cattle, the age corresponds to 6 months old. In goats, the age corresponds to 6 months old. In sheep, the age corresponds to 6 months old. In pigs, the age corresponds to 3 months old. In monkeys, the age corresponds to 9 months old. In dogs, the age corresponds to 3 months old. In cats, the age corresponds to 3 months old. In mice, the age corresponds to 4 weeks old. In hamsters, the age corresponds to 4 weeks old. In guinea pigs, the age corresponds to 4 weeks old.

The "3 months old" used when the subject is a human corresponds to 5 days old in rats. Similarly, in cattle, the age corresponds to 2.5 weeks old. In goats, the age corresponds to 2.5 weeks old. In sheep, the age corresponds to 3 weeks old. In pigs, the age corresponds to 2 weeks old. In monkeys, the age corresponds to 3 weeks old. In dogs, the age corresponds to 1.5 weeks old. In cats, the age corresponds to 1.5 weeks old. In mice, the age corresponds to 5 days old. In hamsters, the age corresponds to 5 days old. In guinea pigs, the age corresponds to 5 days old.

The "1 year old" used when the subject is a human corresponds to 21 days old in rats. Similarly, in cattle, the age corresponds to 10 weeks old. In goats, the age corresponds to 10 weeks old. In sheep, the age corresponds to 12 weeks old. In pigs, the age corresponds to 8 weeks old. In monkeys, the age corresponds to 14 weeks old. In dogs, the age corresponds to 6 weeks old. In cats, the age corresponds to 6 weeks old. In mice, the age corresponds to 21 days old. In hamsters, the age corresponds to 21 days old. In guinea pigs, the age corresponds to 21 days old.

### [Examples]

The invention is explained in more detail below referring to Examples, but the invention is not limited to the Examples.

Five castrated male LWD pigs for each experiment group were subjected to the test and kept at a house pigpen of School of Food Industrial Sciences, Miyagi University. The pigs were introduced at the age of 4 weeks, and after a week of habituation, the test was started using the pigs at the age of 5 weeks. As antibacterial agent-free feed as the basal feed, "HP challenge high milk one R", "Hachimantai A mash", "Sanchoku buta hiiku B" or "Sanchoku buta hiiku C" of Zennoh Feed Mills of the Tohoku District Co, Ltd. was used depending on the growth period. A solution obtained by suspending *Bifidobacterium longum* subsp. *infantis* strain M-63 (called "M-63" below) powder in sterile PBS (-) was orally administered to the pigs of the M-63 administration group once a day at the age of 5 to 16 weeks. The dosage of M-63 was 1.0×10¹¹ cfu/60kg/day and was increased step-wisely every week according to the weight increase, and a solution obtained by suspending tapioca starch in an equivalent amount to that of the M-63 powder, instead of the M-63 powder, in sterile PBS (-) was administered to the pigs of the control group.

### <Experiment 1>

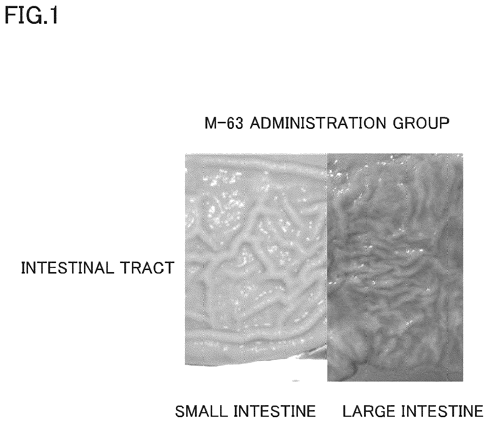

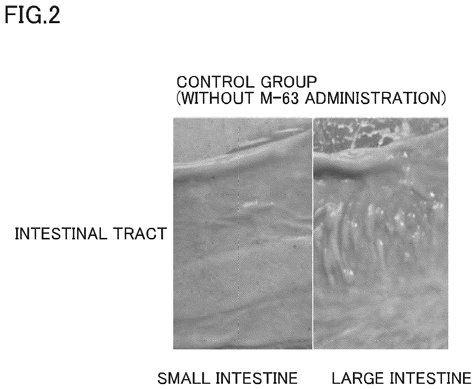

The pigs were dissected at the age of 19 weeks, and the gastrointestinal tracts were taken out. Pictures of the lumina of the intestinal tracts of pigs of the M-63 administration group and the control group are shown in Fig. 1 and Fig. 2, respectively. The numbers of the folds increased in the small intestine and the large intestine of the pigs of the M-63 administration group as compared to those of the pigs of the control group, and the development of the intestinal tract was promoted. It is believed that, in the pigs of the M-63 administration group as compared to the pigs of the control group, intestinal diseases, especially diseases caused from a failure of development of the intestinal tract or a decrease in function, are prevented or treated or that symptoms thereof are relieved.

The tops of the circular folds of the small intestine per 10 cm cross-section in the longitudinal direction were counted, and the average circular fold numbers/cm were determined. The numbers of the tops of the circular folds were counted at three sites per pig. The results are shown in Table 1. The circular fold numbers of the pigs of the M-63 administration group increased significantly as compared to those of the pigs of the control group.

**[Table 1]**

| | Average Circular Fold Number (per 1 cm) | Standard Deviation |
|---|---|---|
| M-63 Administration Group | 0.733 | 0.137 |
| Control Group | 0.507 | 0.144 |

The average body weights of the pigs of the M-63 administration group and the control group at the age of 15 weeks are shown in Table 2. The pigs of the M-63 administration group showed a tendency toward an excellent weight increase as compared to the pigs of the control group.

**[Table 2]**

| | Average Body Weight (kg) | Standard Deviation |
|---|---|---|
| M-63 Administration Group | 72.0 | 32.5 |
| Control Group | 64.3 | 18.0 |

### <Experiment 2>

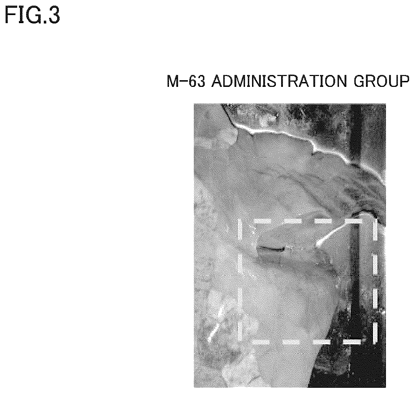

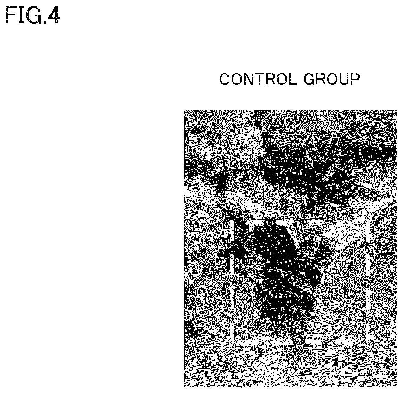

The pigs at the age of 19 weeks were dissected, and the lungs were taken out. Pictures of the right middle lobes and the posterior lobes of the abdominal side of the lungs of pigs of the M-63 administration group and the control group are shown in Fig. 3 and Fig. 4, respectively. A site with reddish brown discoloration and contraction was found in the lung of the pig of the control group (the site surrounded by the dotted line in the figure). The discoloration of the lung tissue is believed to be caused by congestion. It is believed that the lung volume decreased and that the function was lost in a part of the lungs in the pigs of the control group. These findings are typical lesions of mycoplasmal pneumonia of pigs. In the pigs of the M-63 administration group, the lesions were smaller, and lung diseases were prevented or treated or the symptoms thereof were relieved as compared to the pigs of the control group.

The lesion areas in the anterior lobes, the middle lobes, the posterior lobes and the accessory lobes of the lungs were visually observed, and the points according to the lesion areas were added up to calculate the lung lesion score for each individual. The lung lesion scores were calculated referring to Opriessnig T.et al, Vel Pathol,2004 Nov;41(6):624-40 according to the points shown in Table 3. The results are shown in Table 4. The lung lesion scores of the pigs of the M-63 administration group were significantly lower than those of the pigs of the control group.

**[Table 3]**

| Anterior Lobe and Middle Lobe | | | | | | | | | | |
|---|---|---|---|---|---|---|---|---|---|---|
| Lesion Area (%) | 0-10 | 10-20 | 20-30 | 30-40 | 40-50 | 50-60 | 60-70 | 70-80 | 80-90 | 90-100 |
| Point | 0.5 | 1.0 | 1.5 | 2.0 | 2.5 | 3.0 | 3.5 | 4.0 | 4.5 | 5.0 |

| Posterior Lobe and Accessory Lobe | | | | | | | | | | |
|---|---|---|---|---|---|---|---|---|---|---|
| Lesion Area (%) | 0-20 | 20-40 | 40-60 | 60-80 | 80-100 | | | | | |
| Point | 0.5 | 1.0 | 1.5 | 2.0 | 2.5 | | | | | |

**[Table 4]**

| | Lung Lesion Score | Standard Deviation |
|---|---|---|
| M-63 Administration Group | 1.500 | 0.707 |
| Control Group | 3.000 | 0.707 |

### <Experiment 3>

The pigs at the age of 19 weeks were dissected, and the expression levels of cytokine genes in the large intestine tissue were measured. The cytokine expression levels were determined by detecting the mRNA amounts by real-time PCR. As the endogenous control, G3PDH was used. ISOGENII (Nippon Gene Co., Ltd.) was used for RNA extraction, and real-time PCR was conducted using PrimeScript Rtase (Takara Bio Inc.) The primers used for the detection are shown in the sequence listing.

As shown in Tables 5 to 9, the expression levels of IL-6, IL-8, MCP-1, IFN-β and IFN-γ increased in the pigs of the M-63 administration group as compared to those of the pigs of the control group. It was found that, through the administration of M-63, cytokine expression was activated and that the immune function increased.

**[Table 5]**

| | IL-6 Expression Level | Standard Deviation |
|---|---|---|
| M-63 Administration Group | 1.005 | 0.226 |
| Control Group | 0.831 | 0.035 |

**[Table 6]**

| | IL-8 Expression Level | Standard Deviation |
|---|---|---|
| M-63 Administration Group | 0.996 | 0.220 |
| Control Group | 0.842 | 0.036 |

**[Table 7]**

| | MCP-1 Expression Level | Standard Deviation |
|---|---|---|
| M-63 Administration Group | 1.017 | 0.212 |
| Control Group | 0.828 | 0.021 |

**[Table 8]**

| | IFN-β Expression Level | Standard Deviation |
|---|---|---|
| M-63 Administration Group | 0.977 | 0.213 |
| Control Group | 0.806 | 0.038 |

**[Table 9]**

| | IFN-γ Expression Level | Standard Deviation |
|---|---|---|
| M-63 Administration Group | 0.849 | 0.180 |
| Control Group | 0.707 | 0.022 |

CD8 immunostaining of the intestinal tracts of the pigs was conducted according to the following method.
(1) Production of Frozen Blocks
   1. A small intestine or large intestine tissue was washed thoroughly with PBS, and excess water was removed with Kim towel.
   2. Tissue-Tek O.C.T compound (Sakura Finetek Japan Co., Ltd.) was poured into an embedding container to the eight tenths, and the tissue was submerged.
   3. By immersing in liquid nitrogen to freeze instantly, a frozen block was obtained. The frozen block was stored at -80°C until the analysis.
(2) Production of Tissue Sections
   1. The frozen block was cut into a thin piece using a cryostat (Therumo Scientific), and a frozen section was thus produced.
   2. The frozen section was adhered onto an APS-coated glass slide (Matsunami Glass Ind., Ltd.), and thus a frozen tissue section sample was obtained. The tissue section sample was stored at -80°C until the analysis.
(3) Immunofluorescence Staining
   1. TBS-Ca (Tris-Cl 6.06 g, CaCl₂ 0.147 g, NaCl 8.8 g, pH7.6) was poured into a circle drawn with a PAP pen (LIQUID BLOCKER SUPER PAP PEN) around the section, and O.C.T was washed away well.
   2. A 4% paraformaldehyde solution was poured for fixing at room temperature for 15 minutes.
   3. After washing away the fixing solution with TBS-Ca, methanol treatment was conducted at -20°C for 30 minutes, and the solution was replaced again with TBS-Ca.
   4. The glass slide was put in a moisture chamber, and 5% skimmed milk/TBS-Ca was poured into the PAP pen. The glass slide was left still for an hour.
   5. TBS-Ca was poured and washed away after leaving still for three minutes.
   6. The primary antibody was diluted with 5% skimmed milk/TBS-Ca and poured onto the glass slide by the same method as that in 4., and the glass slide was left still at room temperature for an hour.
   7. TBS-Ca was poured and washed away after leaving still for three minutes. This operation was repeated twice.
   8. The secondary antibody was diluted with 5% skimmed milk/TBS-Ca and poured onto the glass slide by the same method as that in 4., and the glass slide was left still at room temperature for 30 minutes.
   9. TBS-Ca was poured and washed away after leaving still for three minutes. This operation was repeated three times.
   10. After dropping Entellan new and sealing with a cover glass, the section was observed and photographed using a fluorescence microscope.

The primary antibody and the secondary antibody are shown in Table 10.

**[Table 10]**

| | Used Antibody | Dilution Rate |
|---|---|---|
| Primary Antibody | Mouse Anti-Bovine CD8 Antibody (Bio-Rad) | 1/50 |
| Secondary Antibody | Alexa Flour 488 goat anti-mouse (Invitrogen) | 1/750 |

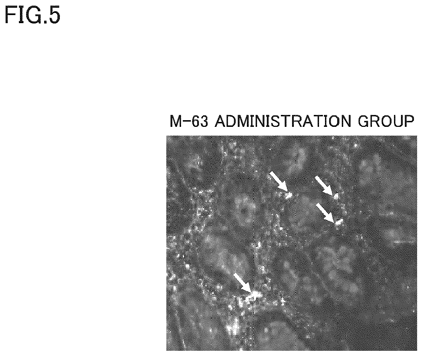

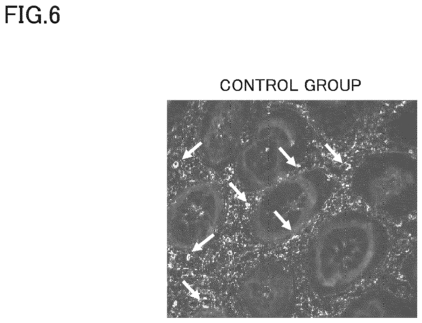

The results of CD8 immunostaining around the small intestine Peyer's patches of the pigs of the M-63 administration group and the control group are shown in Fig. 5 and Fig. 6, respectively. The cells indicated with arrows in the figures are cells with especially strong CD8 expression. Although strong expression of CD8 was observed in the pigs of the control group, the expression of CD8 was suppressed in the M-63 administration group.

Moreover, the fluorescence of a CD8 antibody (abcam, Anti-CD8 alpha antibody [76-2-11] (FITC) (ab24883)) was measured with epifluorescence and DIC microscopes at the same resolution (1040×1388 pixel), and the expression areas of CD8 were measured. The results are shown in Table 11. In the M-63 administration group, the expression areas of CD8 reduced compared to those of the pigs of the control group. CD8 is a marker of killer T cells, and it was shown that there were less cells infected with viruses in the M-63 administration group than those in the control group. It was found that administration of M-63 inhibited viral infections and improved immune function.

**[Table 11]**

| | CD8 Expression Area (pixel) |
|---|---|
| M-63 Administration Group | 22058 |
| Control Group | 102982 |

## Claims

1. A composition for promoting development of the intestinal tract containing a bacterium belonging to *Bifidobacterium longum* subsp. *infantis.*

2. The composition according to claim 1 which promotes formation of a fold in the intestinal tract.

3. A composition for preventing or treating an intestinal disease or relieving a symptom thereof containing a bacterium belonging to *Bifidobacterium longum* subsp. *infantis.*

4. The composition according to claim 3, wherein the intestinal disease is a disease caused from a failure of development of the intestinal tract or a decrease in intestinal tract function.

5. The composition according to any one of claims 1 to 4 which is a food or drink composition.

6. The composition according to any one of claims 1 to 5, wherein the bacterium is *Bifidobacterium longum* subsp. *infantis* M-63 (NITE BP-02623) .

7. The composition according to any one of claims 1 to 6, wherein the bacterium is a living bacterium.

8. The composition according to any one of claims 1 to 7 which further contains a human milk oligosaccharide.

9. The composition according to any one of claims 1 to 8 which is a modified powder milk or a modified liquid milk.

10. An agent for promoting development of the intestinal tract or an agent for preventing or treating an intestinal disease or relieving a symptom thereof containing a bacterium belonging to *Bifidobacterium longum* subsp. *infantis.*

11. Use of a bacterium belonging to *Bifidobacterium longum* subsp. *infantis* for the promotion of development of the intestinal tract or for the prevention or the treatment of an intestinal disease or the relief of a symptom thereof.

12. A bacterium belonging to *Bifidobacterium longum* subsp. *infantis* used for promoting development of the intestinal tract or for preventing or treating an intestinal disease or relieving a symptom thereof.

13. Use of a bacterium belonging to *Bifidobacterium longum* subsp. *infantis* in the manufacture of a composition for promoting development of the intestinal tract or a composition for preventing or treating an intestinal disease or relieving a symptom thereof.

14. A method for promoting development of the intestinal tract or a method for preventing or treating an intestinal disease or relieving a symptom thereof, including a step of administering a bacterium belonging to *Bifidobacterium longum* subsp. *infantis* to a mammal.

15. A composition for improving lung function containing a bacterium belonging to *Bifidobacterium longum* subsp. *infantis.*

16. A composition for preventing or treating a lung disease or relieving a symptom thereof containing a bacterium belonging to *Bifidobacterium longum* subsp. *infantis.*

17. The composition according to claim 16, wherein the lung disease is pneumonia.

18. The composition according to any one of claims 15 to 17 which is a food or drink composition.

19. The composition according to any one of claims 15 to 18, wherein the bacterium is *Bifidobacterium longum* subsp. *infantis* M-63 (NITE BP-02623) .

20. The composition according to any one of claims 15 to 19, wherein the bacterium is a living bacterium.

21. The composition according to any one of claims 15 to 20 which further contains a human milk oligosaccharide.

22. The composition according to any one of claims 15 to 21 which is a modified powder milk or a modified liquid milk.

23. An agent for improving lung function or an agent for preventing or treating a lung disease or relieving a symptom thereof containing a bacterium belonging to *Bifidobacterium longum* subsp. *infantis.*

24. Use of a bacterium belonging to *Bifidobacterium longum* subsp. *infantis* for the improvement of lung function or for the prevention or the treatment of a lung disease or the relief of a symptom thereof.

25. A bacterium belonging to *Bifidobacterium longum* subsp. *infantis* used for improving lung function or for preventing or treating a lung disease or relieving a symptom thereof.

26. Use of a bacterium belonging to *Bifidobacterium longum* subsp. *infantis* in the manufacture of a composition for improving lung function or a composition for preventing or treating a lung disease or relieving a symptom thereof.

27. A method for improving lung function or a method for preventing or treating a lung disease or relieving a symptom thereof, including a step of administering a bacterium belonging to *Bifidobacterium longum* subsp. *infantis* to a mammal.

28. A composition for improving immune function containing a bacterium belonging to *Bifidobacterium longum* subsp. *infantis.*

29. The composition according to claim 28 which regulates production of a cytokine.

30. The composition according to claim 29, wherein the cytokine includes at least one selected from the group consisting of interleukin-6, interleukin-8, monocyte chemotactic protein, interferon-β and interferon-γ.

31. The composition according to claim 28 which inhibits infection with a pathogenic microorganism.

32. The composition according to any one of claims 28 to 31 which is a food or drink composition.

33. The composition according to any one of claims 28 to 32, wherein the bacterium is *Bifidobacterium longum* subsp. *infantis* M-63 (NITE BP-02623) .

34. The composition according to any one of claims 28 to 34, wherein the bacterium is a living bacterium.

35. The composition according to any one of claims 28 to 34 which further contains a human milk oligosaccharide.

36. The composition according to any one of claims 28 to 35 which is a modified powder milk or a modified liquid milk.

37. An agent for improving immune function containing a bacterium belonging to *Bifidobacterium longum* subsp. *infantis.*

38. Use of a bacterium belonging to *Bifidobacterium longum* subsp. *infantis* for the improvement of immune function.

39. A bacterium belonging to *Bifidobacterium longum* subsp. *infantis* used for improving immune function.

40. Use of a bacterium belonging to *Bifidobacterium longum* subsp. *infantis* in the manufacture of a composition for improving immune function.

41. A method for improving immune function, including a step of administering a bacterium belonging to *Bifidobacterium longum* subsp. *infantis* to a mammal.
